# EUROPEAN PATENT SPECIFICATION

(11) **EP 4 134 966 B1**
(45) Date of publication and mention of the grant of the patent: **06.05.2026**
(21) Application number: 21191153.2
(22) Date of filing: 12.08.2021
(51) Int. Cl.: G16H 20/40, G16H 30/40, G16H 40/20, G16H 40/67, G16H 50/20, G16H 70/20, A61B 5/00, A61B 5/11

(54) **OPERATING ROOM VIDEO ANALYTIC SYSTEMS AND METHODS**
OPERATIONSSAALVIDEOANALYTIKSYSTEME UND -VERFAHREN
SYSTÈMES ET PROCÉDÉS D'ANALYSE VIDÉO DE SALLE D'OPÉRATION

(43) Date of publication of application: 15.02.2023
(73) Proprietor: Baxter Medical Systems GmbH + Co. KG, 07318 Saalfeld (DE)
(72) Inventor: DALBERT, Heinz-Hermann, 07318 Saalfeld (DE); WOLFE, Gene, 07318 Saalfeld (DE)
(74) Representative: Reddie & Grose LLP

(56) References cited:
- KR-B1- 102 195 825
- US-A1- 2020 188 047

## Description

### Technical Field

The present invention relates to the field of video analytics systems and methods for use in operating rooms.

### Background

More and more different technologies are being used in operating rooms to aid medical professionals and improve patient care and outcomes. However, integrating these technologies is difficult, as different tools, equipment and systems may be incompatible or require different inputs and outputs, presenting a challenge when trying to maximise the effectiveness of an operating room environment. Furthermore, many tools, devices and equipment also generate data. This data is frequently device and manufacturer specific, further hindering integration. For example, for surgery utilising a robotic surgical instrument to perform portions of the surgery, the robotic surgical instrument needs to know the precise location of the operating room table, and/or the patient relative to that operating table. Positional data may be output by the operating room table, but this is in the form of changes in a step motor configured to move the table. This positional data provides little benefit and cannot be used by, for example, a robotic surgical instrument, unless it has been calibrated prior to use and localised within the operating room. This is a time consuming process, made more undesirable by the fact that an operating room may host many different surgical procedures, each requiring different equipment, and hence requiring new localisation and calibration before each use.

KR102195825B1 relates to a surgery guide system through a notification function and a method thereof, and more particularly, to a system for guiding a surgical position, a drug dose and the like required during a surgical procedure through a notification function that uses light, sound, haptics (vibrations) or the combination of the same by photographing a surgical site of a patient placed on an operating table, a surgical procedure, surgical tools and the like, and comparing and analyzing a photographed image with a reference image according to the gender, body type, surgery type of the patient, and a method thereof. The surgery guide system comprises: a guide signal output part; a guide information monitoring part; and a notification generation part. The document discloses neither determining a position of the operating table from image data using image recognition techniques, nor determining a position of a patient relative to the operating table.

### Summary of Invention

The invention is defined by the appended claims.

### Summary of the Disclosure

According to an aspect of the present disclosure, a method of monitoring a patient on an operating room table is provided. The method comprises receiving image data captured by at least one image capture device; and determining a position of the patient relative to the operating table in dependence on the image data.

Determining a patient position using image data means that the method does not require any specific operating table, and as such can be employed easily to a wide variety of situations. Furthermore, the method can be used with existing operating tables and even with future tables. In addition, as the system is remote from the operating table, it does not compromise patient outcomes or the effectiveness of an operating table as an operating table.

Determining a position of the patient relative to the operating table in dependence on the image data comprises determining a position of the patient from the image data; determining a position of the operating table from the image data; and determining the position of the patient relative to the operating table based on the position of the patient and the position of the operating table. Determining positions of the patient and the operating table allows an accurate position of the patient to be determined, and means that the position of the operating table and patient relative to other objects need not be known. For example, as the position of the operating table is determined, it does not matter if the position moves between different procedures or the like.

Determining the position of the operating table based on the image data means that the type of operating table used does not matter, and that the method can easily be used with existing or future operating tables. In particular, no calibration or other information about the operating table is needed. The position of the operating table is determined using image recognition techniques. The use of image recognition techniques can allow the method to be automatically implemented by a computer or computer system.

The step of determining a position of the patient relative to the operating table may comprise determining a first position of the patient relative to the operating table and determining a second position of the patient relative to the operating table, the second position being determined at a time later than the first position. The method further comprises determining if the patient has moved on the operating table by comparing the first position and the second position. Determining a first and second position allows the position of the patient to be monitored, and for it to be determined if the patient has moved. This can help prevent undesired events from occurring that could be detrimental to patient outcomes, such as a patient sliding, slipping or falling from an operating table.

Optionally, the method further comprises outputting an alert if it is determined that the patient has moved on the operating table. Outputting an alert in this situation means that the problem can be rectified and appropriate action can be taken to ensure that patient outcomes are not compromised. The alert may be output if the patient has moved by at least a threshold amount on the operating table. Outputting an alert if the patient has moved by at least a threshold amount ensures that the method does not output an unnecessary number of alerts, and only outputs an alert when necessary, for example to prevent a patient sliding, slipping or falling from an operating table.

Optionally, the alert is output if the patient has moved out of a predefined region on the operating table. A patient moving out of a predefined region on the operating table, e.g., a central region, can be indicating of the patient may be sliding, slipping or falling from an operating table. Accordingly, outputting an alert if this is detected can prevent these adverse incidents from occurring.

Optionally, determining a position of the patient on an operating table comprises determining a plurality of sub-positions of the patient based on the image data, the sub-positions of the patient being positions of parts of the patient's body. Determining the positions of the different parts of a patient's body can allow just the parts of interest to be monitored, and also means that the posture, pose or position of the patient can be determined and also monitored.

An alert may be output if it is determined that the patient is not in an acceptable position and/or if it is determined that the patient is in an unacceptable position based upon the plurality of sub-positions of the patient. An acceptable and unacceptable position (e.g. a pose or posture), can be detrimental to patient outcomes, for example by causing sores or bruising, and may not be noticed by a medical professional during surgery due to, for example, the patient being beneath a sheet or other object, or simply because their attention is on the surgery. Accordingly, detecting the position, pose or posture of the patient using image data may improve patient outcomes.

The alert may be output if the patient is not in an acceptable position and/or if it is determined that the patient is in an unacceptable position for at least a threshold period of time. Outputting an alert if the patient is not in an acceptable position and/or if it is determined that the patient is in an unacceptable position for at least a threshold period of time ensures that the method does not output an unnecessary number of alerts, and only outputs an alert when necessary. Different threshold periods of time may be set for different positions. This again further reduces the number of unnecessary alerts output whilst also ensuring that patient outcomes are not compromised by enabling very bad situations to be quickly rectified.

The parts of the patient's body corresponding to sub-positions of the patient's body may include one or more of: head; arms; hands; legs; feet; torso; and abdomen. Identifying the sub-positions of these parts of the patient effectively allow the position, pose or posture of the patient to be determined and monitored.

Preferably, determining a position of the patient on an operating table comprises determining the position of a patient underneath a sheet. Determining the position of a patient underneath a sheet means that even if part of the patient is covered then the method is still effective, and further when the patient is covered then medical professionals will likely be less aware of the position of the patient, meaning that the method is particularly helpful.

The position of the patient underneath a sheet may be determined at least in part from image data captured by a thermal imaging and/or infrared camera. The use of a thermal camera and/or an infrared camera can effectively determine the position of a patient underneath a sheet because many sheets will not obstruct the infrared light given off by a patient's body, whilst they may block visible light that can be seen by the human eye.

Optionally, the step of determining a position of the patient underneath a sheet comprises determining a surface of the sheet above the operating table and determining the position of the patient underneath the sheet based on the surface. As a sheet laying over the patient will often be distorted by the body of the patient, it is possible to determine the position of the patient based on the surface of the sheet, in particular by determining the three-dimensional shape of the surface of the sheet. From this surface, the patient's position can be deduced.

Optionally, the surface of the sheet above the operating table is determined at least in part from image data captured using one or more of radar, LIDAR, ultrasound devices, ultrasound, and time-of-flight imaging.

The position of the patient is identified using image recognition techniques. The use of image recognition techniques can allow the method to be automatically implemented by a computer or computer system.

The method may further comprise the step of capturing, by one or more image capture devices, the image data. Optionally, the image data comprises one or more of visible spectrum data, thermal or infrared data, depth or time-of-flight data, radar data, LIDAR data, ultrasound data, and stereoscopic image data.

According to a tenth aspect of the present disclosure, a system for monitoring a patient on an operating room table is provided. The system comprises one or more image capture devices configured to capture image data and one or more computing devices configured to carry out the method of the ninth aspect.

Optionally, the one or more image capture devices are one or more of visible spectrum cameras, thermal or infrared cameras, depth or time-of-flight cameras, radar, LIDAR, ultrasound devices, and stereoscopic imaging cameras.

According to an eleventh aspect of the present disclosure, a computer program is provided. When run on one or more computing device, the computer program is configured to cause the one or more computing devices to perform the method of the ninth aspect.

According to a twelfth aspect of the present disclosure, a non-transitory memory having stored thereon the computer program of the eleventh aspect is provided.

### Brief Description of the Drawings

The disclosure will be further described, by of example only, with reference to the accompanying drawings, in which:
Figure 1 illustrates a system according to aspects of the disclosure;
Figure 2 illustrates a method according to a first aspect of the disclosure;
Figure 3 illustrates a method according to a first aspect of the disclosure;
Figure 4 illustrates a method according to a fifth aspect of the disclosure;
Figure 5 illustrates a method according to a ninth aspect of the disclosure;
Figure 6 illustrates a method according to a ninth aspect of the disclosure;
Figure 7 illustrates a method according to a thirteenth aspect of the disclosure;
Figure 8 illustrates a method according to a thirteenth aspect of the disclosure;
Figure 9 illustrates a method according to a thirteenth aspect of the disclosure;
Figure 10 illustrates a method according to a seventeenth aspect of the disclosure;
Figure 11 illustrates a method according to a seventeenth aspect of the disclosure; and
Figure 12 illustrates a method according to a seventeenth aspect of the disclosure.

### Detailed Description of the Invention

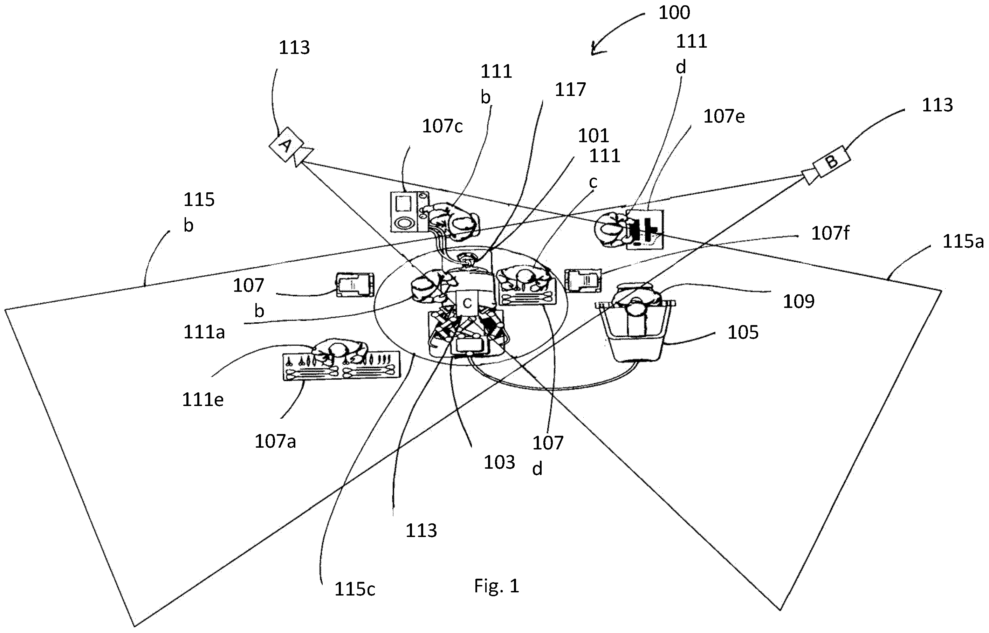

A system according to aspects of the present disclosure will be described with respect to Figure 1.

Figure 1 illustrates an operating room 100 in which a system according to aspects of the present disclosure is implemented. Operating room 100 comprises an operating table 101 supporting a patient 117. In the example illustrated in Figure 1, the patient 117 is undergoing thoracic surgery with a robotic surgical instrument 103, though it will be appreciated that the invention is not limited to this type of surgery. The robotic surgical instrument 103 is controlled through a surgeon console 105, operated by surgeon 109.

In addition to the operating table 101 and the robotic surgical equipment 103, the operating room 100 comprises a number of other pieces of equipment 107. In this example, the operating room further comprises a first table 107a having surgical tools thereon, a vision cart 107b, an anaesthesiologist's cart 107c, a second table 107d having further surgical tools thereon, a nurse's workstation 107e, and a laparoscopic tower 107f.

In addition to the surgeon 109, a number of other medical professionals are located in the operating room 100. In this example, a first assistant 111a and a second assistant 111c are present, an anaesthesiologist 111b is present, a nurse 111d is present and a surgical technician 111e is present.

It should be noted that the equipment and medical professionals listed above and illustrated within the operating room 100 in Figure 1 are exemplary and do not limit the invention. The equipment and medical professionals required will be, for example, determined by the surgery that is being performed.

Image capture devices 113 configured to capture image data of within the operating room 100 are also provided. In Figure 1, three such image capture devices 113 are provided: image capture device A; image capture device B; and image capture device C. These are provided such that they can capture image data. The image data is representative of a field of view within the operating room. However, it should be noted that the image capture devices themselves do not have to be located within the operating room. For example, they may be disposed behind a wall that does not obstruct the view of the image capture device (e.g. behind a glass wall for a camera operating in the visual spectrum).

The three image capture devices 113 each have a different field of view of within the operating room 100. The field of view of image capture device A is shown by cone 115a, the field of view of image capture device B is shown by cone 115b, and the field of view of image capture device C is shown by ellipse 115c. It will be appreciated that image capture devices A and B are located to the sides of the operating room looking inwards, whilst image capture device C is located above the operating table 101 looking downwards.

It can be seen that the fields of view 115 of each of the image capture devices 113 all cover a region where the patient 117 is being operated on with the robotic surgical instrument 103 by the surgeon 109. This is an example of a region of interest, and according to aspects of the present disclosure the image capture devices 113 are configured such that their fields of view 115 include that region of interest. Typically, a region of interest will be a region wherein surgery is taking place, as illustrated in Figure 1, though other regions, volumes and objects may also be "regions of interest".

The image capture devices 113 are positioned such that they are likely to have an unobstructed view of a region of interest, and multiple image capture devices 113 can help to compensate if the field of view of one image capture device 113 is obstructed. For example, as can be seen in Figure 1, if image capture device A were obstructed by anaesthesiologist's cart 107c then image capture devices B and C would still provide an unobstructed view of the region of interest.

The number and the position, as well as the type, of image capture devices may vary depending upon the use case of the present disclosure. For example, whilst three image capture devices 113 are illustrated in Figure 1, with specific positions, other embodiments of the present disclosure may have fewer (e.g., one or two) or more (e.g., four, five, ten, etc.) image capture devices. Furthermore, these may be positioned differently to the image capture devices 113 illustrated in Figure 1.

The image capture devices may also be static or moveable. In the case that they are moveable, this may be in between procedures (e.g. they can be moved on trollies or the like) or during a procedure (e.g., they may be attached to a manoeuvrable arm). Moveable image capture devices can be advantageous for being adaptable to different types of surgery that may require different fields of view (e.g., of different regions of interest). Image capture devices moveable during a procedure can advantageously help to overcome an obstruction in the field of view of the image capture device by enabling the field of view of the image capture device to be changed.

A number of different types of image capture devices may be used that capture different types of image data. A non-exhaustive list of different types of image data that could be captured by the image capture device includes visible spectrum data, thermal or infrared data, depth or time-time-of-flight data, radar data, LIDAR data, and stereoscopic image data. To capture these types of data, the image capture devices may be visible spectrum cameras, thermal or infrared cameras, depth or time-of-flight cameras, radar, LIDAR, or stereoscopic imaging cameras. Other types of image data and image capture devices than those listed herein may also be used.

Different image types of image capture device may be provided in different locations. For example, in Figure 1, image capture device A may be a visible spectrum camera at a first location, whilst image capture device B may be an infrared camera at a second location. In some cases, an image capture device may capture multiple types of image data. This may be because the image captured device comprises multiple sub-image capture devices of different types that are connected or otherwise joined together such that they act as one unit or because the sensor or other image capturing module is sensitive to or configured to capture more than one type of image data. For example, an image capture device may be configured to capture both visible spectrum image data and infrared image data, or to capture both visible spectrum image data and time-of-flight image data.

Aspects of the present disclosure provide a number of different methods that can be implemented using the systems described herein, such as that illustrated in Figure 1.

One aspect of the present disclosure provides a method of monitoring objects within an operating room, and in particular involves a method of overcoming obstructions of an image capture device.

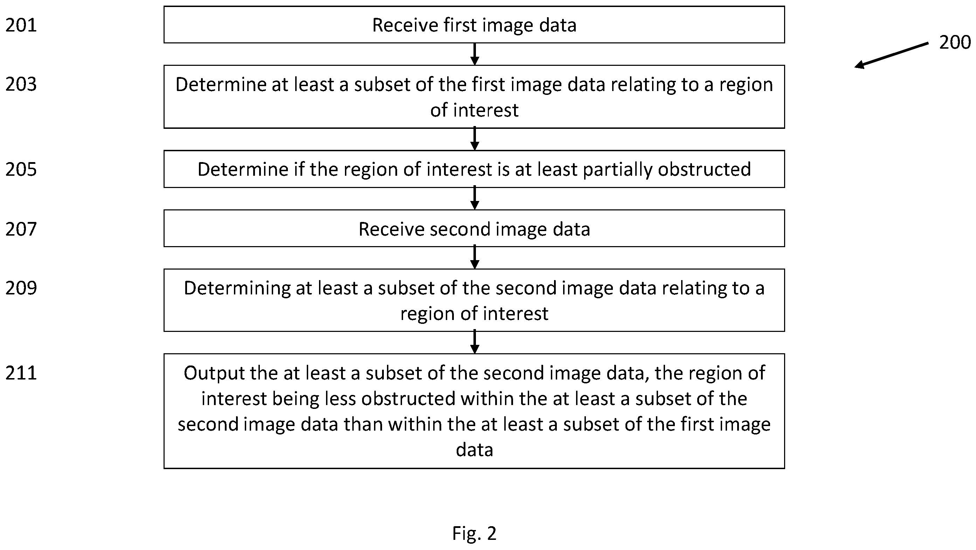

A flowchart of the method 200 is illustrated in Figure 2. Method 200 begins at step 201 with receiving first image data. The first image data is image data captured by at least one image capture device. The image data may be captured by a single image capture device or multiple image capture devices.

At step 203, at least a subset of the first image data is determined. The at least a subset of the first image data (which may therefore comprise all the captured image data or only some of the captured image data) relates to a region of interest within the operating room. The region of interest may be identified by a medical professional, and subsequently determined using image recognition techniques. Alternatively, the region of interest may be identified automatically in dependence on the type of surgical procedure, or in dependence on a predefined list; e.g. the list may comprise surgical tools; patient body parts; and medical equipment.

The region of interest may be static region of interest or it may not be static. That is to say, the region of interest may move throughout a surgical procedure. For example, the region of interest may be an object (such as a piece of equipment or a surgical tool) or a person, or a volume around such an object or person, and the region of interest may move with the object or person. As another example, the region of interest may be a region in which the surgical procedure is to be performed, such as a torso region of a patient.

At step 205, it is determined whether the region of interest within the operating room is at least partially obstructed within the subset of the first image data. This is done based on the first image data, that is, it is determined if the region of interest is obstructed in dependence of the first image data. It may be determined that the region of interest is obstructed, for example, if, after an unobstructed view of the region of interest is obtained, it is detected that another object has moved in front of the region of interest thus obscuring it. In another case, it may be determined that the region of interest is obstructed if certain objects cannot be identified within the image data. For example, if it is known that a trocar should be within the region of interest but a trocar cannot be identified in the subset of the first image data, then it may be determined that the region of interest is obscured. Other techniques may also be used.

For example, determining if the region of interest within the operating room is at least partially obstructed may be done by obtaining a model (such as a 3D volumetric representation) of the region of interest or of an object in the region of interest. This model can then be deformed. For example, vectors defining a 3D volumetric representation of the object or region of interest are projected to a plane of the first image data. The object, as identified in the first image data, and the deformed model can then be compared to generate a relational score between the two. If this score is below a threshold value, then it may be determined that the region of interest is at least partially obstructed.

Upon determining that the region of interest is at least partially obscured, the method moves to step 207. At step 207, second image data captured by the at least one image capture device is received. Then, at step 209, at least a subset of the second image data is determined (which may comprise all the captured image data or only some of the captured image data), the at least a subset of the second image data relating to the region of interest. At step 211, the at least a subset of the second image data is output; the at least a subset of the second image data relates to the region of interest and the region of interest is less obstructed within the at least a subset of the second image data compared to within the at least a subset of the first image data.

The second image data received at step 207 may be some combination of image data of the same type as the first image data captured by a different image capture device at a different position to the image capture device that captured the first image data, image data of the same type as the first image data captured by the same image capture device at a different position to the image capture device that captured the first image data (e.g., the image captured device moved between capturing the first and second image data), and image data of a different type to the first image data captured by the same or a different image capture device at the same or a different position as when the first image data was captured.

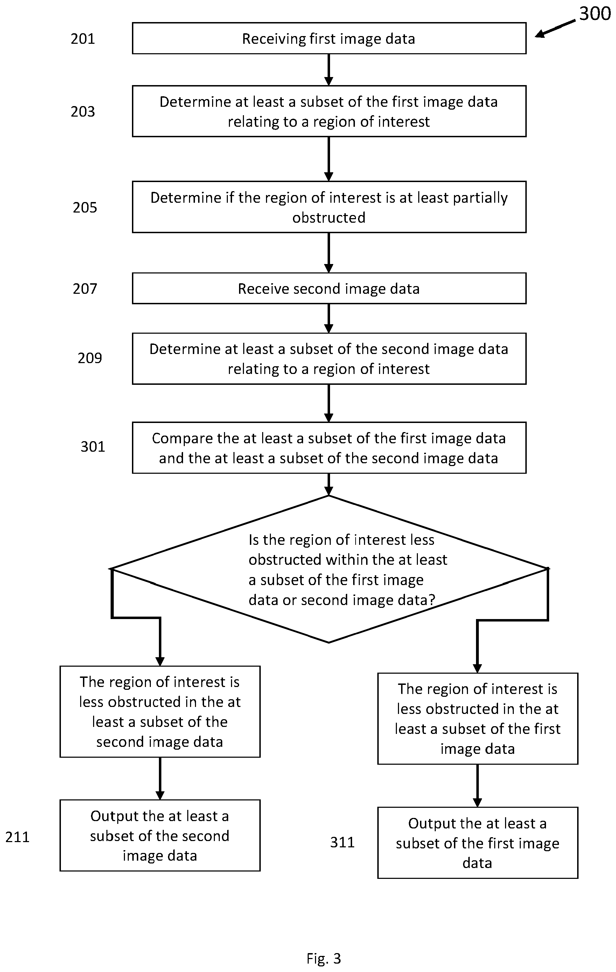

In some cases, the subset of the first image data determined at step 203 is compared with the subset of the second image data determined at step 209. Such a method is illustrated in Figure 3.

The first steps of Figure 3 are the same as those illustrated in Figure 2. Specifically, steps 201 to 209 are again performed in the method of Figure 3 as they were in Figure 2, and so the discussion of these steps is not repeated.

After step 209, in the method of Figure 3, the at least a subset of the first image data is compared with the at least a subset of the second image data, at step 301. This is done to determine whether the region of interest is less obstructed in the at least a subset of the first image data or whether the region of interest is less obstructed in the at least a subset of the second image data.

If it is determined that the region of interest is less obstructed within the at least a subset of the second image data, then step 211 is performed, as in the method of Figure 2, and the at least a subset of the second image data is output. However, if it is determined that the region of interest is less obstructed within the at least a subset of the first image data, then step 311 is performed instead of step 211, and the at least a subset of the first image data is output. In this manner, it can be ensured that the image data corresponding to the best view of the region of interest is output, and that any obstruction to an image capture device is negated as far as possible. It should be noted that whilst the method is described with respect to two sets of image data, the principles may be applied to any number of image data sets. For example, three, four, five, ten, or another number of image data sets could be received, a subset of each image data set relating to a region of interest could be determined, and all of these subsets of image data could be compared to determine for which subset the region of interest is least obstructed, so that this subset of image data can be output.

The comparison between the first image data and the second image data to determine in which the region of interest is more obstructed may be achieved by generating a relational score from the at least a subset of the second image data in the same manner as described above for the first image data and then comparing the relational scores for the first image data or the second image data.

The first image data and the second image data, used in both the methods of Figure 2 and of Figure 3, may be captured by the same image capture device but from a different position. In this case, the methods comprise the step of moving the at least one image capture device to a new position such that the field of view of the region of interest from the at least one image capture device is no longer obstructed or is obstructed to a lesser extent. In this case, the method may also comprise determining the new position in order that it provides a field of view that is less obstructed that the original field of view. In this case, the method may comprise the step of determining a volume occupied by the region of interest based on the first image data, determining a three-dimensional position of the obstruction object based on the first image data; determining a second position from which the field of view of the region of interest will not be obstructed or will be obstructed to a lesser extent, and moving the at least one image capture device from the first position to the second position.

Moving the image capture device may comprise a translation of the image capture device (e.g., by moving on wheels) or a rotation or pivoting of the image capture device, amongst other movements. In some cases, the image capture device may be attached to an articulated arm, such as a robotic arm, that can move to provide a desired translation and/or rotation of the image capture device.

The movement of the image capture device may be along a movement trajectory, which is a trajectory from the first position to the second position. The movement trajectory can be generated using a look up table, based on the location of the region of interest, the location of the first position and the three-dimensional position of the obstruction object. A candidate position to give an unobstructed view may be determined from the look up table, and a trajectory may be determined based on the first position and the candidate position, for example using the look up table.

For example, turning to Figure 1, an image capture device is at a first position A, represented by image capture device A. The region of interest is centred around the patient 117, and so the view that the image capture device has of the region of interest, from position A, is obstructed by the anaesthesiologist's cart 107c and the anaesthesiologist 111b. The anaesthesiologist's cart 107c and the anaesthesiologist 111b are therefore identified as being obstruction objects, and their positions are determined, or at least estimated, based on the image data captured by the image capture device from position A. Based on this information, as well as the location of position A and the region of interest, a look up table is consulted. This process identifies a position B, represented in Figure 1 by image capture device B, as a candidate position that will have an unobstructed view, and a trajectory is determined from position A to position B.

In this example, the process is iterative, although it is should be understood that this is not essential. When iterative, the process comprises moving the image capture device towards the candidate position along the movement trajectory from the first position to a second position along the movement trajectory. A score is then compared between the first position and the second position, the score representing an amount by which the region of interest is obstructed. For example, the relational scores discussed above may be calculated at the first and second positions, and if they indicate that region of interest is less obstructed from the second position then the next iteration may continue to move the image capture device along the movement trajectory. If the scores indicate that, after moving to the second position, the region of interest is more obscured than from the first position, an alternative trajectory can be computed for the next iteration of the method.

Continuing the example discussed above with reference to Figure 1 wherein a trajectory is determined from position A to position B, as the image capture device approaches position B it may be determined that the region of interest around the patient 117 is obstructed by nurse 111d and the nurse's workstation 107e. Accordingly, a new trajectory may be determined, for example taking a position C, represented in Figure 1 by image capture device C, as the candidate position. The image capture device may then be moved along this new trajectory. This process continues until an acceptable view of the region of interest is achieved.

Alternatively, or in addition, to moving an image capture device to overcome an obstruction, multiple image capture devices can be used that capture different image data. This could be image data of the same type from a different position, image data of a different type from the same position, or image data of a different type from a different position.

For example, in such a case, when applied to the method of Figure 2 or Figure 3, the first image data may be captured by a first image capture device and the second image data may be captured by a second image capture device. In this case, the at least one image capture devices is at least two image capture devices. The second image device may be determined based on the first image data. For example, a volume of the region of interest may be determined based on the first image data, as may a three-dimensional position of an obstruction object that is obstructing the image of the first image capture device. It can then be determined whether image data of the region of interest from one or more of the at least two image capture devices (other than the first image capture device) will be less obstructed based on the volume of the region of interest, the three-dimensional position of the obstruction object, and known positions of the image capture devices. Upon determining that the image data of the region of interest from one of the other image capture devices will be less obstructed than the subset of the first image data relating to the region of interest from the first capture device, then that image capture device from which the image data will be less obstructed can be determined as the second image capture device.

By way of an example, consider the anaesthesiologist's cart 107c and the anaesthesiologist 111b shown in Figure 1. These may obstruct the field of view of a region of interest, centred around the patient 117, of the image capture device A. If the image capture device A is the first image capture device, then it may be determined that image capture device B will not have a field of view obstructed by the anaesthesiologist's cart 107c and the anaesthesiologist 111b, and so image capture device B may be determined as the second image capture device. Image data from image capture device B may, therefore, be output instead of image data from image capture device A.

In this example, the first image capture device and the second image capture device are located in different positions, such that they have different fields of view. This can enable a second image capture device to capture unobstructed, or less obstructed, image data relating to the region of interest than a first image capture device. However, additionally or alternatively, the first image capture device and the second image capture device may be different types of image capture device, configured to produce different types of image data. This can mean that the first and second image capture devices may be in the same position (and so have the same field of view) but the image data generated by the first image capture device may be obstructed whilst the image data generated by the second image capture device may not be. This is particularly the case when the obstructing object is transparent to a certain form of light or electromagnetic radiation but not another form. For example, different wavelengths of electromagnetic radiation can pass through different materials without being blocked. In some implementations, the first image capture device may be a visible spectrum camera whilst the second image capture device may be an infrared camera. Thus, whilst one type of image capture device may have its view obstructed by an obstructing object, a different type of image capture device may not, even with the same field of view. It should be noted that whilst the first and second image capture devices are described as separate devices, they may be integrated with one another so as to form a single unit.

In both the case where the image capture device is moved to overcome an obstruction and/or where multiple image capture devices are used to overcome an obstruction, the image data captured from the different positions and/or the different image capture devices may be combined. In this case, the method further comprises the steps of combining the first image data and the second image data to generate composite image data and outputting the composite image data. The composite image data relates to the region of interest, with the region of interest being less obstructed in the composite image data than in either the first image data or the second image data.

The combining of the image data can be achieved in a number of ways. For example, if different types of image data are captured by different types of image capture device, but from substantially the same position, then the image data in one of the first or second sets of image data that relates to an obstruction object can be removed, and replaced with the equivalent image data that is unobstructed from the other image capture device. For example, if one image capture device is a visible spectrum camera and another is an infrared camera, if an object is obstructing the view of the visible spectrum camera but not the infrared camera (e.g. a sheet which blocks visible light but not infrared), then image data from the infrared camera can be used to replace the portions of the image data from the visible spectrum camera that are obstructed.

In the case that the positions of the two image capture devices from which image data is being combined are different, then some or all of the image data may need to be morphed or projected before it can be combined. For example, based on first image data, a three-dimensional representation of an object in the region of interest can be created, and this can then be projected into the plane of the second image data and then used to replace a portion of the second image data that is obstructed. In order to project the data in this manner, the image data may be interpolated or extrapolated so that gaps in the available image data can be overcome. Artificial intelligence techniques such as machine learning may be used for this purpose, and may utilise reference images of the identified object from one or more angles to assist in projecting the image and fill in any missing portions in the image data.

In some cases, missing portions in the data can be overcome using three-dimensional models of an object in the region of interest. Continuing the above example, after the object in the region of interest is identified from the first image data, a three-dimensional model can be obtained and this can be projected into the plane of the second image data. This can help to provide visible spectrum image data from other forms of data. For example, if an object is identified using infrared image data, a three-dimensional model of this object can be mapped to the object so that it can be viewed as if it were captured with a visible spectrum image capture device. Hence, the composite image data may comprise both portions of the first image data, the second image data, and computer-generated data.

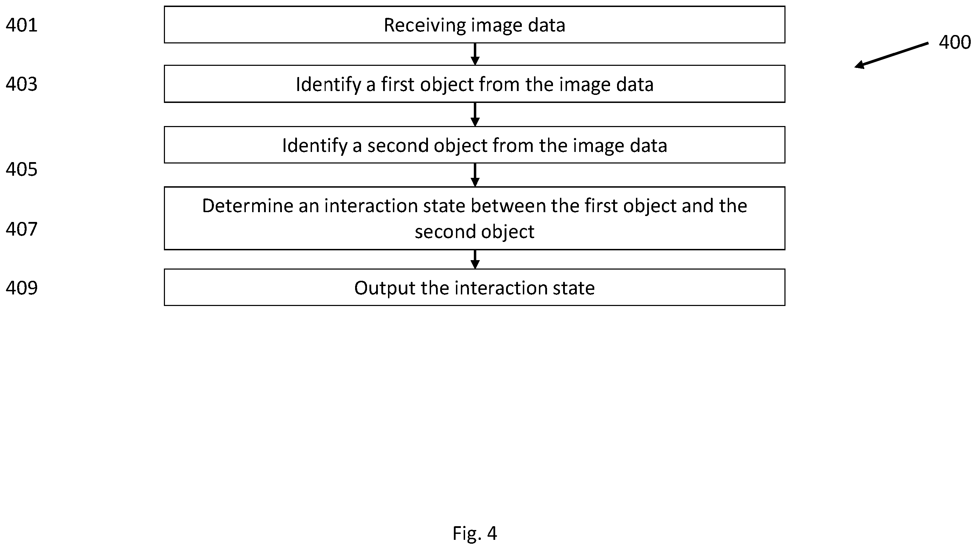

Another aspect of the present disclosure described herein provides another method of monitoring objects within an operating room. This method is described with respect to Figure 4.

Method 400 begins at step 401 with receiving image data captured by at least one image capture device. The image capture device and the image data may be of any type discussed herein.

At step 403, a first object is identified from the image data, and at step 405 a second object is identified from the image data. The first and second objects are identified from the image data using image recognition techniques of the type well known to those skilled in the art.

Subsequently, at step 405, an interaction state between the first object and the second object is determined. The interaction state can include a compatibility state (e.g. compatible, not compatible), a relative positional state (e.g. a distance between the objects, potential collision risk), and a current interaction (e.g. coupled, coupling, uncoupled, uncoupling) etc.

Once the interaction state has been determined at step 407, the interaction state is output at step 409. This could include outputting to another part of a computer program for subsequent use, outputting to another device, and outputting for a medical professional to observe.

The interaction state may be based on the volumes of the first object and the second object. In this case, the method may further include determining a volume occupied by the first object based on the image data and determining a volume occupied by the second object based on the image data. This can help to provide information as to whether the objects are compatible. In some cases, objects may be determined as not being compatible if they cannot fit within or around each other. For example, the first and second object may be determined to not be compatible if the first object cannot fit within or around the second object. An example use case of such a method would be if the first object were a trocar and the second object were a laparoscope. The method would determine whether the trocar were of an appropriate size for the laparoscope. If it were determined that the laparoscope would not fit within the trocar, then it would be determined that the laparoscope and trocar were not compatible and an alert could be output to a surgeon. Another exemplary situation would be determining whether a large piece of equipment such as a robotic surgical implement were compatible with an operatic table based on whether the operating table could fit within a defined region of the robotic surgical implement.

In some cases, the interaction state may be a state of impending collision or not. That is, determining the interaction state may comprise determining whether the first object and the second object will collide. This can be done by determining a velocity of the first object from the image data and determining a velocity of the second object from the image data. The velocities may be determined relative to an external reference frame (e.g., the operating room) or relative to one another. In this case outputting the interaction state may comprise outputting an alert if it is determined that the first and the second object will collide. This could be in the form of an audible and or visual warning, such as a message on a screen, a warning light and a verbal warning.

Alternatively or additionally, outputting the interaction state may comprise outputting a command configured to adjust the velocity of either or both of the first object and the second object to prevent the first object and the second object from colliding. For example, if one or both of the first object and the second object are being controlled by a computer, a command may be sent to the computer that, when executed by the computer, causes the computer to adjust the velocity of the first and or second object. For example, the velocity may be adjusted to zero, i.e., the first and or the second object may be caused to stop moving.

The interaction state, in particular the alert or the adjust velocity command as discussed above, may be output a predetermined time before a collision is predicted to occur. The time until a collision is predicted to occur may be based on the determined velocities of the first and second object. For example, if the velocity of the first object is determined to be 0 m/s and the velocity of the second object is determined to be 0.1 m/s in the direction of the first object, and the distance between the objects is 0.5 m, it may be determined that the objects will collide in 5 seconds. The predetermined time may be 1 second before a collision, 2 seconds before collision, 5 seconds before collision, or another time. If the output interaction state is a warning to enable a medical professional to take action then preferably the predetermined time is longer (e.g. 10 seconds) to give the medical professional enough time to react to prevent collision. If the output interaction state is a command to a computer to prevent the collision then the predetermined time may be shorter (e.g. 1 second or less) as the computer does not need as much time to execute the command.

In determining whether a collision will occur, the volume occupied by each object may be considered. A collision may be considered to be imminent when it is predicted that the volumes occupied by each object will overlap based on current velocities. The volumes may be a compound volume that precisely matches the size and shape of the object, or it may be a simple and/or regular volume that encompasses the object. The latter may require less computing power to implement. In some cases, the volume may be one of a parallelepiped (e.g. a cuboid), a sphere, or a cylinder. An appropriate shape may automatically be assigned to an identified object based on the captured image data.

As well some or all of position, velocity and volume information about the first and second objects, other data may also be used to determine if a collision will occur. In particular, information may be received from one or both of the first and second objects (or from a device or computer controlling them) regarding a trajectory or future movement path of the objects. For example, information may be provided about how far an object will move in a certain direction, how long it will move for, a path of the object, and/or a final position of the object. This information can be combined with the information derived from the image data to further refine the determination as to whether a collision will occur. For example, whilst two objects may have relative velocities that will result in a collision, information from each object may indicate that the objects will stop moving prior to a collision and so it may be that no collision will occur.

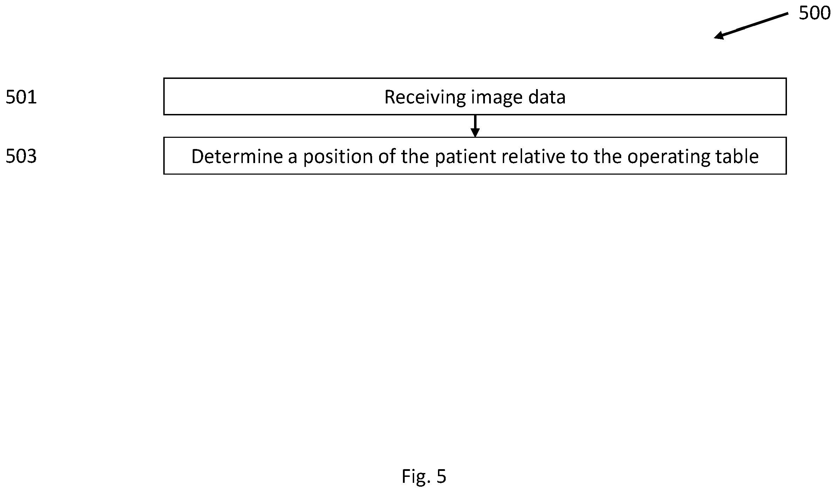

Another aspect of the present disclosure described herein provides a method of monitoring a patient on an operating room table. This method is described with respect to Figure 5.

At step 501 of method 500 illustrated in Figure 5, image data captured by at least one image capture device is received. The image capture device and the image data may be of any type discussed herein.

At step 503, a position of the patient relative to the operating table is determined. In particular, this may be a position of the patient on the operating table. This position is determined based on the image data. For example, a patient can be located in the image data using image recognition techniques and their position subsequently determined. The position of the operating table can be similarly determined using image recognition techniques and then a relative position of the patient on the operating table can be determined. Alternatively, the position of the operating table may be determined based on position data of the operating table received from the operating table or some other source. The position of the patient relative to the operating table can then be determined based on the patient position (determined based on the image data) and the operating table position (determined based on the received position data). The positions determined may be three-dimensional positions.

It should be noted that the positions of the patient and the operating table may be determined relative to some external coordinate system (e.g. relative to the operating room) and compared to generate a relative position, or they can be determined directly relative to one another. For example, an origin can be determined based on the operating table (e.g. a top left hand corner of an operating table could be identified and used as the origin), and then the position of the patient could be determined relative to this.

The position of the patient relative to the operating table can be used to determine if a patient moves. This can be helpful to ensure a patient stays in the correct position such that a robotic surgical implement can operate properly, or to ensure that a patient is not sliding or slipping on the operating table, for example if the operating table is not horizontal.

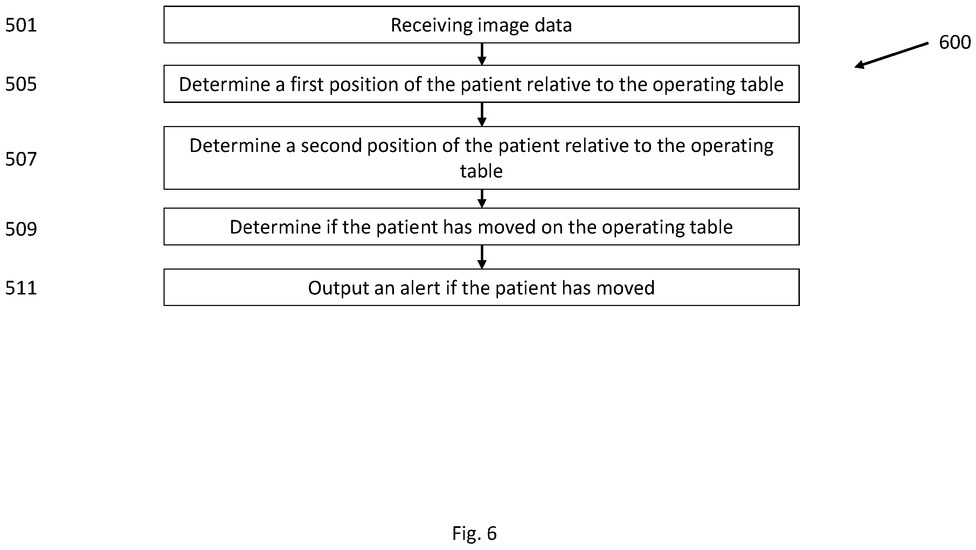

An example of such a method is illustrated in Figure 6. Method 600 begins at step 501 which is the same as in method 500 illustrated in Figure 5. The method then moves to step 505, wherein a first position of the patient relative to the operating table is determined, and then to step 507, wherein a second position of the patient relative to the operating table is determined. The second position is determined at a time after the first position was determined. For example, it may be determined 1 second after, 5 seconds after, 30 seconds after, 1 minute after, or some other period of time later. The time difference between recording the first and second positions may be a fixed and/or predetermined value. Again, the first and second positions may, in particular, be positions of the patient on the operating table.

Once the first and second positions have been determined, the method moves to step 509 whereby the first and second positions are compared to determine if the patient has moved on the operating table. If the first and second positions are different, then it may be determined that the patient has moved, whereas if the first and second positions are the same, then it may be determined that the patient has not moved.

A threshold difference may be used to determine whether the patient has moved. For example, whilst the two positions may not be exactly the same the difference may be sufficiently small (i.e. below a threshold value) that the difference is considered insignificant and it is determined that the patient has not moved. The threshold value could be a relative value (e.g. a certain percentage of a height or length of the patient, which may be determined from the image data or otherwise input) or an absolute value (e.g. a given number of centimetres). Different thresholds may be used for different directions of movement (i.e. a larger threshold may be used for movement along the length of an operating table whilst a smaller threshold may be used for movement across a width of an operating table) and for different patients and types of surgery or procedure.

Alternatively, or in addition, it may be determined that a patient has moved on the operating table if the patient has moved out of a predefined region on the operating table. For example, if the second position is determined to be a position outside of such a predefined region then it may be determined that the patient has moved, whereas if the second position is a position within the predefined region then it may be determined that the patient has not moved. The predefined region may be a region that it is considered to be safe for the patient to be in. For example, it may be a central region such that it is considered that the patient is not at risk of slipping, sliding or falling of the operating table. Such a position could be, for example, the region of the operating table that is 5cm or more from an edge of the operating table. Different predefined regions may be used for different patients and types of surgery or procedure.

In some cases, both a threshold and a predefined region may be used. For example, it may be determined that a patient has moved if the second position is different from the first position by a threshold amount or the second position is a position outside a predefined region. In other cases, it may be determined that a patient has moved if the second position is different from the first position by a threshold amount and the second position is a position outside a predefined region.

If it is determined that the patient has moved, then the method can proceed to step 511. At step 511 an alert is output. This could be in the form of an audible and or visual warning, such as a message on a screen, a warning light, and a verbal warning. For example, it may be configured to alert a medical professional that they need to attend to the position of the patient. Additionally or alternatively, the alert may be in the form of a computer command configure to cause a piece of equipment to perform a certain action, such as causing an operating table to adjust its position (e.g. level itself).

The first and second positions may represent a variety of different things. For example, the positions could be a single point or a plurality of discreet points. Such points could be an estimated centre of mass determined based on the image data or another reference point, such as a top of the head or points of specific facial features (e.g., eyes). Alternatively, the positions may be areas or volumes, e.g., an area or volume occupied by a patient, which could also be determined from the image data. The volume occupied by a patient could be represented by a cuboid that the patient fits within, for example, the smallest such cuboid.

First and second positions may also correspond to positions of one or more parts of the patient's body. For example, the first and second position may each comprise a plurality of sub-positions that are determined based on the image data. These sub-positions may be three-dimensional positions of parts of the patient's body. Such parts could be, for example, a head, torso, abdomen, arms, legs, feet, hands, etc. The position of these parts of a body may be any of the types of position described in the preceding paragraph.

From the determined sub-positions of parts of a patient's body, a position of the patient can be determined. For example, it can be determined if the patient is in a supine or prone position, as well as a position of the arms, legs and head. It can be determined if the position of the patient is an acceptable position or not. For example, some positions may increase the risk of complications or patient discomfort, particularly if the position is maintained for an extended period of time.

If it is determined that the patient is not in an acceptable position or if it is determined that the patient is in an unacceptable position then an alert is output. This may be determined by comparing the position of a patient against a list of acceptable positions and determining that the patient is not in an acceptable position if the determined position of the patient does not match a position on the list of acceptable positions. Similarly, it may be determined by comparing the position of a patient against a list of unacceptable positions and determining that the patient is in an unacceptable position if the determined position of the patient matches a position on the list of unacceptable positions. In either case, an alert may be output. This could be in the form of an audible and or visual warning, such as a message on a screen, a warning light, and a verbal warning.

As noted above, not being in an acceptable position, or being in an unacceptable position, may be undesirable if the patient remains in such a position for an extended period of time. Therefore, the alert may be output if the position is maintained for at least a threshold period of time, i.e., if the patient is not in an acceptable position, or in an unacceptable position, for a threshold period of time. The threshold period of time may be different for different patients or groups of patients (e.g., certain classes of patient may be more at risk of certain complications arising due to being in an unacceptable position and so the threshold period of time for these patients may be shorter), and it may also vary with position. For example, some positions may be deemed less acceptable, worse, or more dangerous than other positions, and so the threshold period of time may be shorter for these positions.

In some cases, the patient's body may be fully or partially covered by a sheet or other item. In this case, the position of the patient underneath the sheet or other item may be determined. This may be done based on the three-dimensional shape formed by the sheet or other item using image recognition techniques and/or depth information, time-of-flight information, radar, LIDAR, ultrasound and stereoscopic information captured by the at least one image capture device. In addition, or alternatively, the position may be determined using thermal imaging or infrared image data. This can be effective, particularly for thin sheets, at determining a patient position whilst using relatively little computational power. Such techniques can be applied to any of the methods of monitoring a patient position on an operating table described herein, such as those of Figures 5 and 6.

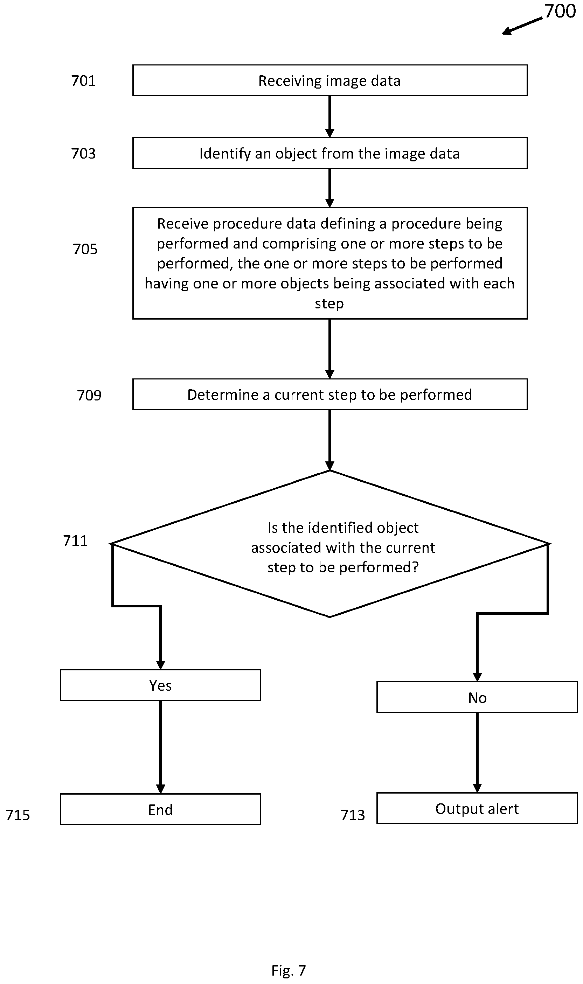

Another aspect of the present disclosure described herein provides a method of monitoring objects within an operating room. Such a method is described with respect to Figure 7.

Method 700 begins at step 701, wherein image data captured by at least one image capture device is received. The image capture device and the image data may be of any type discussed herein.

The method then proceeds to step 703, which comprises identifying an object from the image data. The object is identified using image recognition techniques well known to those skilled in the art.

Step 705 comprises receiving procedure data. The procedure data defines a procedure being performed (e.g. an operation or type of surgery, such as bypass surgery) and comprises one or more steps to be performed. That is, the procedure data comprises information regarding the different steps that are performed during the procedure. The procedure data also comprises information regarding one or more objects that are associated with each step. For example, the procedure data may include a list of the surgical tools that are required for each step. It should be noted that step 705, whilst illustrated as subsequent to steps 701 and 703 in Figure 7, may in fact be performed prior to, or simultaneously with, steps 701 and/or 703.

At step 709, a current step to be performed is determined. That is, it is determined which step of the procedure is currently being performed. This may be done based on the image data (e.g. certain objects and object states may be recognised) or based on some other input. For example, a medical professional may input the current step into a computer.

At step 711, the method determines whether the object identified at step 703 is one of the one or more objects associated with the current step to be performed. For example, the method determines if a surgical tool identified at step 703 is one of the surgical tools that are required for the current step of the procedure or not. This may be done, for example, by comparing the identified object to a list of the objects associated with each step.

If it is determined that the identified object is not one of the one or more objects associated with the current step to be performed, then an alert is output at step 713. This could be in the form of an audible and/or visual warning, such as a message on a screen, a warning light, and a verbal warning. This can ensure that an incorrect object (e.g. incorrect surgical tool) is not inadvertently used, which could lead to complications for the patient.

On the other hand, if the identified object is one of the one or more objects associated with the current step to be performed, then the method ends at step 715.

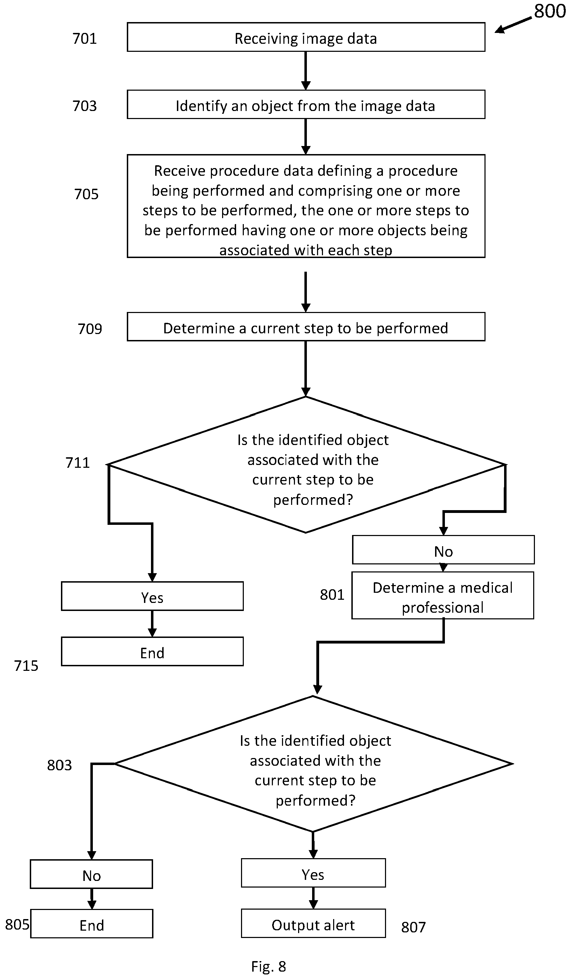

In some cases, it is determined not just whether an incorrect object is present, but that an incorrect object is being used by a medical professional. Such a method is illustrated in Figure 8.

Method 800 begins with steps 701 to 711, which are the same as those described above with respect to method 700 of Figure 7. Similarly, if at step 711 the identified object is determined to be associated with the current step to be performed, the method again ends at step 715.

However, unlike method 700 of Figure 7, in method 800, if the identified object is determined to not be an object associated with the current step to be performed, then the method proceeds to step 801. At this step, a medical professional is determined, e.g. based on the image data. It is noted that this step may be performed prior to, or simultaneously with any of steps 701 to 711.

Then, at step 803, it is determined whether the object is being used by a medical professional. I f it is determined that the identified object is not being used by a medical professional then the method ends at step 805, whilst if it is determined that the identified object is being used by a medical professional then the method outputs an alert at step 807. This could be in the form of an audible and/or visual warning, such as a message on a screen, a warning light, and a verbal warning. Hence, the alert is output in method 800 if the identified object is being used by a medical professional and is not an object associated with a current step being performed.

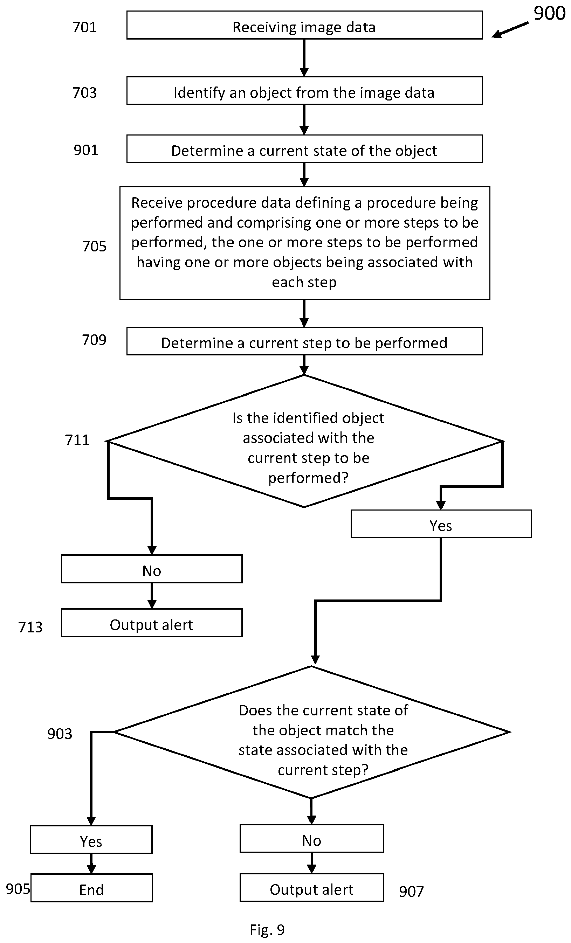

In some cases, in addition or alternatively to determining if the identified object is being used by a medical practitioner, a state of the object can be used to determine whether an alert is output. Figure 9 illustrates an example of such a case.

Steps 701 to 711 of method 900, illustrated in Figure 9, are the same as those performed in methods 700 and 800. Method 900 also comprises step 901, at which a current state of the identified object is determined, based on the image data. This step is illustrated as being between steps 703 and 705 in Figure 9, though it will be appreciated that it may be performed before, after, or simultaneously with any of steps 701 to 711.

The state of the object may be an initial state of the object (e.g., the state the object is in at the beginning of the procedure). The initial state of each object associated with a procedure may be preset. For example, an initial state of a surgical tool may be a "sterile" state, indicating that that surgical tool is sterile. Examples of possible states, which may be initial states or otherwise, include a "sterile" state, an "in use" state, a "used" state, an "open" state, a "closed" state, a "moving" state, a "stationary" state, an "idle" state, a "locked" state, an "unlocked" state, and a "paused" state.

At step 711, if it is determined whether the identified object is not one of the one or more objects associated with the current step to be performed, then an alert is output at step 713. This could be in the form of an audible and/or visual warning, such as a message on a screen, a warning light. and a verbal warning. On the other hand, if the object is not one of the objects associated with the current step then the method moves to step 903.

At step 903 it is determined whether the current state of the object matches the state associated with the current step to be performed. If the current state of the object does match the state associated with the current step to be performed then the method ends at 905. If the current state of the object does not match the state associated with the current step to be performed then an alert is output at 907. This could be in the form of an audible and/or visual warning, such as a message on a screen, a warning light, and a verbal warning.

An exemplary application of such a method is to ensure that a surgeon is using a sterile surgical tool in a medical procedure. For example, the first step of a procedure may be to create an incision in a patient using a scalpel. Therefore, a scalpel will be an object associated with this step. It is important that the scalpel is sterile, and so the state of the object associated with this step will be a "sterile" state, which may be determined from the image data. If the scalpel is identified at step 703 and if the state that is determined at step 901 is a "sterile" state, then at step 903 it will be determined that the current state of the scalpel matches the state associated with that step of the procedure. However, if the state is a different state, such as if it is determined that the scalpel is in a "used" state, then the current state of the scalpel will be determined to not match the state associated with that step at step 903. In this case, an alert is output to warn the surgeon that they are about to use a non-sterile scalpel.

In the method of any of Figures 7 to 9, the current state may be stored in memory, as may any changes to the current state (e.g., a transition from "sterile" to "used"), with timestamps associated with the state or the change in state. A record may be generated comprising this information for each object used in a procedure, allowing for information regarding the procedure to be reviewed at a later time. The record may be stored as part of an electronic medical record of the patient, and/or a case protocol for the particular surgery.

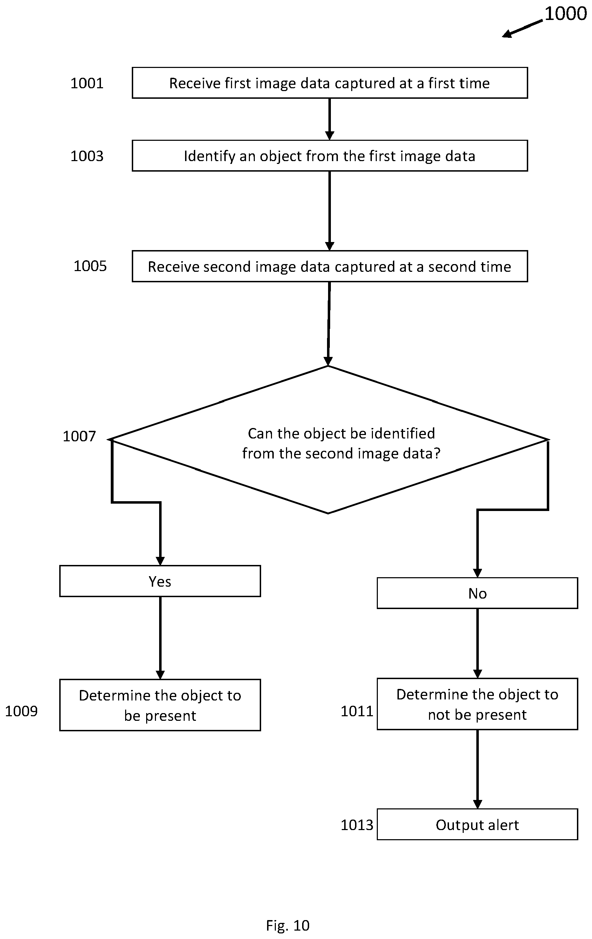

Another aspect of the present disclosure described herein provides a method of monitoring objects within an operating room. Such a method is described with respect to Figure 10.

The method 1000 begins at step 1001, whereby first image data is received. The first image data is captured at a first time, by at least one image capture device. The image capture device and the image data may be of any type discussed herein.

At step 1003, an object is identified from the first image data. The object is identified using image recognition techniques.

At step 1005, second image data is received. The second image data is captured at a second time, being after the first time, by the at least one capture device. That is, the second image data is image data captured after the first image data, and so corresponds to a later point in time.

Then, at step 1007, it is determined whether the object can be identified from the second image data. As with step 1003, this may be done using image recognition techniques. If the object can be identified then it is determined that the object is present at the second time, and the method ends at step 1009. However, if the object cannot be identified, then, at step 1011, it is determined that the object is not present, or least not visible, within the operating room at the second time, and so at step 1013 an alert is output. This could be in the form of an audible and/or visual warning, such as a message on a screen, a warning light, and a verbal warning.

The first time can be a start or beginning of a procedure, and the second time may be an end of a procedure. In this case, one particular use of the method 1000 is to ensure that no surgical tools are missing at the end of the procedure. This can be especially useful in ensuring that no surgical tools or other items are left inside a patient at the end of surgery, which can cause complications for the patient and may require further surgery to remove.

In some cases, however, an object may be present in the operating room but not visible by at least one of the image capture devices due to an obstruction blocking the view of the image capture device. Such a situation can be overcome by methods 1100 and 1200.

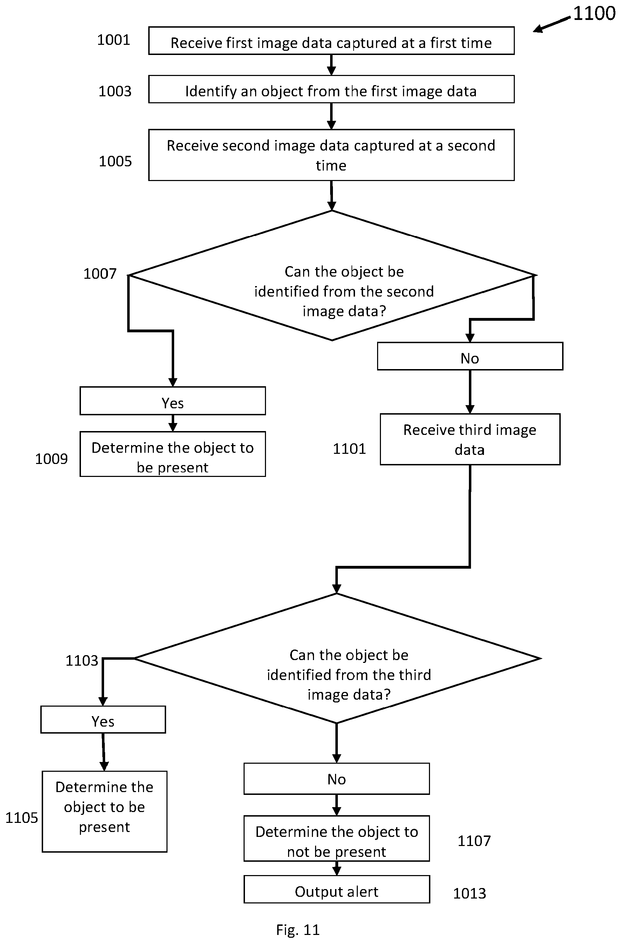

Firstly, method 1100 is described with respect to Figure 11. Method 1100 begins with steps 1001 to 1007, as with method 1000 of Figure 10. As with method 1000, when it is determined that the object can be identified in the second image data at step 1007, it is then determined that the object is present within the operating room at step 1009. However, if it is determined that the object cannot be identified within the second image data, then the method moves to step 1101.

At step 1101, third image data is received. The third image data corresponds at least in part to image data from a different source than the second image data. That is, the second image data is captured by a first set of the at least one image capture devices whilst the third image data is captured by a second set of the at least one image capture devices, wherein the second set includes at least one image capture device not in the first set. For example, the second image data could be received from image capture device A in Figure 1, whilst the third image data could be received from image capture device B. Preferably, the at least one image capture device of the second set that is not in the first set is in a different position to the image capture devices of the first set, so as to have a different field of view of the operating room, or captures image data of a different type.

The method then proceeds to step 1103, where it is determined whether the object can be identified in the third image data. If the object can be identified, it is determined that the object is present in the operating room at step 1105, whilst if the object cannot be identified it is determined that the object is not present in the operating room at step 1107. In this case, as with method 1000, an alert may be output at step 1013. This could be in the form of an audible and/or visual warning, such as a message on a screen, a warning light, and a verbal warning.

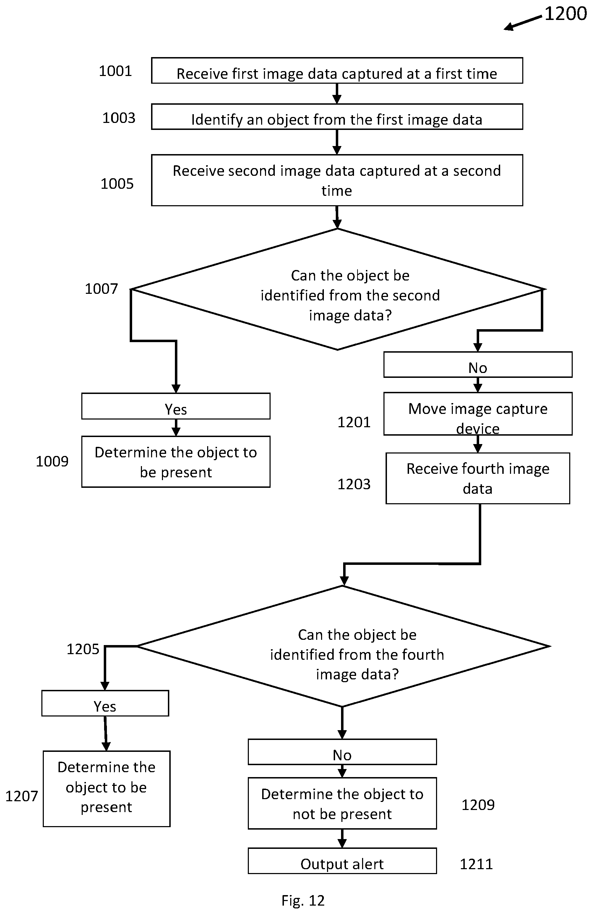

Another method, which may be performed additionally with or alternatively to method 1100, is method 1200, described in Figure 12. Method 1100 begins with steps 1001 to 1007, as with method 1000 of Figure 10, though the second image data received at step 1005 is captured by a first image capture device from a first position. As with method 1000, when it is determined that the object can be identified in the second image data at step 1007, it is then determined that the object is present within the operating room at step 1009. However, if it is determined that the object cannot be identified within the second image data, then the method moves to step 1201.

At step 1201, the first image capture device is moved to a second position. In particular, it is moved such that the field of view of the first image capture device is changed. That is, the field of view of the first image capture device after being moved at step 1201 is different to the field of view of the first image capture device prior to being move at step 1201. Then, at step 1203, fourth image data is received. The fourth image data is captured by the first image capture device at the second position.

Once the fourth image data is received, at step 1205 it is determined whether the object can be identified from the fourth image data. If it can be, then at step 1207 it is determined that the object is present in the operating room. If it cannot be then it is determined at step 1209 that the object is not present in the operating room. In this case, an alert may be output at step 1013. This could be in the form of an audible and/or visual warning, such as a message on a screen, a warning light, and a verbal warning.

Reference to positions, directions (such as up and down), and velocities herein, unless otherwise specified, may be relative to a coordinate system of the operating room or to another object within the operating room. When using a coordinate system of the operating room, an object described as having a velocity of zero will be stationary with respect to the walls, floor and ceiling of the operating room. The origin of this coordinate system may be chosen as any convenient point, and so references to a "position" should be construed as a reference to a position in the operating room with respect to the walls, floor and ceiling of the operating room but should be interpreted as being limiting on the origin against which the position is measured. An appropriate origin may be selected by the skilled person when implementing the invention described herein on a case by case basis. For example, the origin may be taken as a point on the floor of the operating room, or a central point of the operating room (which may be in a position other than on a floor or wall of the operating room, for example, in mid-air). In each case, unless otherwise specified, a position may be a three-dimensional position of an object within the operating room or a two dimensional position of an object with respect to some surface in the operating room (e.g., a floor or an operating table) or with respect to the image data (e.g., a two dimensional position in the image itself).

Throughout this specification, the identification of objects from image data may be performed using image recognition techniques known to the skilled person, unless otherwise specified. For example, convolutional operations to detect edges and textures is used to process the raw image into a set of masks and regions. Optionally an atlas based representation tree is applied for the determination of an object within frame. This tree shows dependence information about where an object "should" be with respect to another object - and allows the algorithm to better identify an object, for example with an increased confidence. For example, dependence information may link a patient with being on an operating table, or a surgical tool being on a tray.

Such image recognition techniques can include the use of artificial intelligence techniques, such as machine learning and in particular deep learning and neural networks. Image recognition techniques can include various techniques for recognising and identifying, classifying, detecting, and tagging objects from images. The image recognition may require the training of a neural network using a training set, which may comprise images of objects that are intended to be recognised. Such a neural network may be continuously updated and re-trained. It will be appreciated that the methods described herein may be performed using a wide range of appropriate algorithms and techniques known in the art, and the method is hence not limited in this regard.

The image data referred to herein, unless otherwise specified, may comprise image data in the form of a single image or video frame or may be a series of consecutive or nonconsecutive images or video frames. These may be analysed individually or separately, or they may be analysed together. Furthermore, the methods herein, whilst described as a series of discreet steps, may be performed continuously on a continuous stream of image data, for example in the form of a video. That is, the methods may continuously analyse the video (e.g., they may analyse each video frame, or a subset of the video frames, such as every tenth frame) and perform the method continuously on the video (e.g., on each selected frame).

The identification of objects performed in the methods described herein may include, unless otherwise specified, the identification of whole objects, parts of an object, pieces of equipment, parts or regions of a surface such as a wall, floor or table, people, parts of people, surgical tools and objects, organs, amongst others. This is a non-exhaustive list.

The identification may be a classification of an object as a certain class of object (e.g., an object may be classified as a person, or as a class of person, such as a surgeon or a patient), or it may further include a specific identification of the object as a unique entity (e.g., the person may be identified as an individual person, such as Joe Blogs). In the case of equipment and objects, these may be identified based on a serial number or other identifying tag or feature (e.g., a visual marking, such as a QR code). In the case of people, these may be identified based on a biometric property such as a face or retina, or they may be identified based on a tag or mark associated with them, such as a marking (e.g., a QR code) on an item of clothing.

In some cases, it may be preferable to avoid the capture of personal or identifying data due to legal or regulatory requirements, and so as to preserve the privacy and confidentiality of an individual. In this case, certain portions of image data may be excluded from analysis, such as a face. This can ensure that personal information is not collected or processed. This may be a default case, but the option may be available for an individual to opt-in to personal identification. This can be advantageous so as to be able to link information captured during a procedure to the individual or to personalise settings of the system. For example, a surgeon may prefer a particular arrangement of image capture devices and so upon identifying the surgeon the system may automatically configure this arrangement of image capture devices. In another case, image data, such as a video, may be recorded and saved and linked with a patient for review by their doctor or other medical practitioner at a later date. However, in other cases, the image data collected and used in the methods herein may not be saved or stored, only being kept for as long as is required to perform the relevant processing upon it as required by the method. This can prevent personal data from being accidentally or deliberately lost or misused.

The methods described herein may be performed on a single computing device (on one or more processors), or across multiple computing devices in a distributed system. Such a distributed system may be connected by a local area network or a wide area network. The methods may also take advantage of cloud computing services to perform one or more steps of the method.

## Claims

1. A computer implemented method of monitoring a patient (117) on an operating table (101), the method comprising:
receiving (501) image data captured by at least one image capture device (113); and
determining (503) a position of the patient (117) relative to the operating table (101) in dependence on the image data;
wherein determining (503) a position of the patient (117) relative to the operating table (101) in dependence on the image data comprises:
determining a position of the patient (117) from the image data;
determining a position of the operating table (101) from the image data; and
determining the position of the patient (117) relative to the operating table (101) based on the position of the patient (117) and the position of the operating table (101);
wherein the position of the patient (117) and the operating table (101) is determined using image recognition techniques.

2. The method of claim 1, wherein determining a position of the patient (117) relative to the operating table (101) comprises:
determining (505) a first position of the patient (117) relative to the operating table (101); and
determining (507) a second position of the patient (117) relative to the operating table (101), the second position being determined at a time later than the first position;
wherein the method further comprises determining (509) if the patient (117) has moved on the operating table (101) by comparing the first position and the second position.

3. The method of claim 2, wherein the method further comprises outputting (511) an alert if it is determined that the patient (117) has moved on the operating table (101).

4. The method of claim 3, wherein the alert is output if the patient (117) has moved by at least a threshold amount on the operating table (101) and/or wherein the alert is output if the patient (117) has moved out of a predefined region on the operating table (101).

5. The method of any of claims 1 to 4, wherein determining a position of the patient (117) on an operating table (101) comprises determining a plurality of sub-positions of the patient (117) based on the image data, the sub-positions of the patient (117) being positions of parts of the patient's body.

6. The method of claim 5, wherein an alert is output if it is determined that the patient (117) is not in an acceptable position and/or if it is determined that the patient (117) is in an unacceptable position based upon the plurality of sub-positions of the patient (117).

7. The method of claim 6, wherein the alert is output if the patient (117) is not in an acceptable position and/or if it is determined that the patient (117) is in an unacceptable position for at least a threshold period of time, wherein optionally different threshold periods of time are set for different positions.

8. The method of any of claims 5 to 7, wherein the parts of the patient's body corresponding to sub-positions of the patient's body may include one or more of: head; arms; hands; legs; feet; torso; and abdomen.

9. The method of any of claims 1 to 8, wherein determining a position of the patient (117) on an operating table (101) comprises determining the position of a patient (117) underneath a sheet, wherein optionally the position of the patient (117) underneath a sheet is determined at least in part from image data captured by a thermal imaging and/or infrared camera.

10. The method of claim 9, wherein the position of the patient (117) underneath a sheet comprises:
determining a surface of the sheet above the operating table (101); and
determining the position of the patient (117) underneath the sheet based on the surface.

11. A system for monitoring a patient (117) on an operating table (101) comprising:
one or more image capture devices (113) configured to capture image data; and
one or more computing devices configured to carry out the method of any of claims 1 to 10.

12. A computer program which, when run on one or more computing device, is configured to cause the one or more computing devices to perform the method of any of claims 1 to 10.

13. A non-transitory memory having stored thereon the computer program of claim 12.

## Patentansprüche

1. . Computerimplementiertes Verfahren zur Überwachung eines Patienten (117) auf einem Operationstisch (101), wobei das Verfahren Folgendes umfasst:
Empfangen (501) von Bilddaten, die von mindestens einer Bildaufnahmevorrichtung (113) erfasst wurden; und
Bestimmen (503) einer Position des Patienten (117) in Bezug auf den Operationstisch (101) in Abhängigkeit von den Bilddaten;
wobei das Bestimmen (503) einer Position des Patienten (117) relativ zu dem Operationstisch (101) in Abhängigkeit von den Bilddaten Folgendes umfasst:
Bestimmen einer Position des Patienten (117) aus den Bilddaten;
Bestimmen einer Position des Operationstisches (101) aus den Bilddaten; und
Bestimmen der Position des Patienten (117) in Bezug auf den Operationstisch (101) auf der Grundlage der Position des Patienten (117) und der Position des Operationstisches (101);
wobei die Position des Patienten (117) und des Operationstisches (101) mithilfe von Bilderkennungstechniken bestimmt wird.

2. . Verfahren nach Anspruch 1, wobei das Bestimmen einer Position des Patienten (117) in Bezug auf den Operationstisch (101) Folgendes umfasst:
Bestimmen (505) einer ersten Position des Patienten (117) in Bezug auf den Operationstisch (101); und
Bestimmen (507) einer zweiten Position des Patienten (117) in Bezug auf den Operationstisch (101), wobei die zweite Position zu einem späteren Zeitpunkt als die erste Position bestimmt wird;
wobei das Verfahren weiter das Bestimmen (509) umfasst, ob sich der Patient (117) auf dem Operationstisch (101) durch Vergleichen der ersten Position und der zweiten Position bewegt hat.

3. . Verfahren nach Anspruch 2, wobei das Verfahren weiter das Ausgeben (511) eines Alarms umfasst, wenn festgestellt wird, dass der Patient (117) sich auf dem Operationstisch (101) bewegt hat.

4. . Verfahren nach Anspruch 3, wobei der Alarm ausgegeben wird, wenn der Patient (117) sich um mindestens eine Schwellenmenge auf dem Operationstisch (101) bewegt hat und/oder wobei der Alarm ausgegeben wird, wenn der Patient (117) sich aus einem vordefinierten Bereich auf dem Operationstisch (101) bewegt hat.

5. . Verfahren nach einem der Ansprüche 1 bis 4, wobei das Bestimmen einer Position des Patienten (117) auf einem Operationstisch (101) das Bestimmen einer Vielzahl von Unterpositionen des Patienten (117) auf der Grundlage der Bilddaten umfasst, wobei die Unterpositionen des Patienten (117) Positionen von Körperteilen des Patienten sind.

6. . Verfahren nach Anspruch 5, wobei ein Alarm ausgegeben wird, wenn bestimmt wird, dass sich der Patient (117) nicht in einer akzeptablen Position befindet, und/oder wenn bestimmt wird, dass sich der Patient (117) auf der Grundlage der Vielzahl von Unterpositionen des Patienten (117) in einer inakzeptablen Position befindet.

7. . Verfahren nach Anspruch 6, wobei der Alarm ausgegeben wird, wenn sich der Patient (117) nicht in einer akzeptablen Position befindet, und/oder wenn bestimmt wird, dass sich der Patient (117) für mindestens einen Schwellenzeitraum in einer inakzeptablen Position befindet, wobei wahlweise unterschiedliche Schwellenzeiträume für verschiedene Positionen eingestellt sind.

8. . Verfahren nach einem der Ansprüche 5 bis 7, wobei die Teile des Körpers des Patienten, die Unterpositionen des Körpers des Patienten entsprechen, einen oder mehrere der folgenden einschließen können:
Kopf, Arme, Hände, Beine, Füße, Rumpf und Bauch.

9. . Verfahren nach einem der Ansprüche 1 bis 8, wobei das Bestimmen einer Position des Patienten (117) auf einem Operationstisch (101) das Bestimmen der Position eines Patienten (117) unter einem Laken umfasst, wobei wahlweise die Position des Patienten (117) unter einem Laken mindestens teilweise aus von einer Wärmebild- und/oder Infrarotkamera erfassten Bilddaten bestimmt wird.

10. . Verfahren nach Anspruch 9, wobei die Position des Patienten (117) unter einem Laken Folgendes umfasst:
Bestimmen einer Oberfläche des Lakens oberhalb des Operationstisches (101); und
Bestimmen der Position des Patienten (117) unter dem Laken auf der Grundlage der Oberfläche.

11. . System zur Überwachung eines Patienten (117) auf einem Operationstisch (101), umfassend:
eine oder mehrere Bildaufnahmevorrichtungen (113), die zum Erfassen von Bilddaten ausgebildet sind; und
eine oder mehrere Rechenvorrichtungen, die zur Durchführung des Verfahrens nach einem der Ansprüche 1 bis 10 ausgebildet sind.

12. . Computerprogramm, das, wenn es auf einer oder mehreren Rechenvorrichtungen ausgeführt wird, so ausgebildet ist, dass es die eine oder mehrere Rechenvorrichtungen veranlasst, das Verfahren nach einem der Ansprüche 1 bis 10 durchzuführen.

13. . Nichtflüchtiger Speicher, der das Computerprogramm nach Anspruch 12 darin aufweist.

## Revendications

1. Procédé mis en œuvre par ordinateur de surveillance d'un patient (117) sur une table d'opération (101), le procédé comprenant :
la réception (501) de données d'image capturées par au moins un dispositif (113) de capture d'images ; et
la détermination (503) d'une position du patient (117) par rapport à la table d'opération (101) en fonction des données d'image ;
dans lequel la détermination (503) d'une position du patient (117) par rapport à la table d'opération (101) en fonction des données d'image comprend :
la détermination d'une position du patient (117) à partir des données d'image ;
la détermination d'une position de la table d'opération (101) à partir des données d'image ; et
la détermination de la position du patient (117) par rapport à la table d'opération (101) sur la base de la position du patient (117) et de la position de la table d'opération (101) ;
dans lequel la position du patient (117) et de la table d'opération (101) est déterminée en utilisant des techniques de reconnaissance d'images.

2. Procédé selon la revendication 1, dans lequel la détermination d'une position du patient (117) par rapport à la table d'opération (101) comprend :
la détermination (505) d'une première position du patient (117) par rapport à la table d'opération (101) ; et
la détermination (507) d'une deuxième position du patient (117) par rapport à la table d'opération (101), la deuxième position étant déterminée à un temps postérieur à la première position ;
dans lequel le procédé comprend en outre le fait de déterminer (509) si le patient (117) s'est déplacé sur la table d'opération (101) en comparant la première position et la deuxième position.

3. Procédé selon la revendication 2, dans lequel le procédé comprend en outre la délivrance en sortie (511) d'une alerte s'il est déterminé que le patient (117) s'est déplacé sur la table d'opération (101).

4. Procédé selon la revendication 3, dans lequel l'alerte est délivrée en sortie si le patient (117) s'est déplacé d'au moins une quantité seuil sur la table d'opération (101) et/ou dans lequel l'alerte est délivrée en sortie si le patient (117) s'est déplacé en dehors d'une région prédéfinie sur la table d'opération (101).

5. Procédé selon l'une quelconque des revendications 1 à 4, dans lequel la détermination d'une position du patient (117) sur une table d'opération (101) comprend la détermination d'une pluralité de sous-positions du patient (117) sur la base des données d'image, les sous-positions du patient (117) étant des positions de parties du corps du patient.

6. Procédé selon la revendication 5, dans lequel une alerte est délivrée en sortie s'il est déterminé que le patient (117) n'est pas dans une position acceptable et/ou s'il est déterminé que le patient (117) est dans une position inacceptable sur la base de la pluralité de sous-positions du patient (117).

7. Procédé selon la revendication 6, dans lequel l'alerte est délivrée en sortie si le patient (117) n'est pas dans une position acceptable et/ou s'il est déterminé que le patient (117) est dans une position inacceptable pendant au moins une période de temps de seuil, dans lequel facultativement des périodes de temps de seuil différentes sont définies pour des positions différentes.

8. Procédé selon l'une quelconque des revendications 5 à 7, dans lequel les parties du corps du patient correspondant à des sous-positions du corps du patient peuvent inclure un ou plusieurs parmi :
la tête ; les bras ; les mains ; les jambes ; les pieds ; le torse ; et l'abdomen.

9. Procédé selon l'une quelconque des revendications 1 à 8, dans lequel la détermination d'une position du patient (117) sur une table d'opération (101) comprend la détermination de la position d'un patient (117) sous un drap, dans lequel facultativement la position du patient (117) sous un drap est déterminée au moins en partie à partir de données d'image capturées par une caméra d'imagerie thermique et/ou une caméra infrarouge.

10. Procédé selon la revendication 9, dans lequel la position du patient (117) sous un drap comprend :
la détermination d'une surface du drap au-dessus de la table d'opération (101) ; et
la détermination de la position du patient (117) sous le drap sur la base de la surface.

11. Système de surveillance d'un patient (117) sur une table d'opération (101) comprenant :
un ou plusieurs dispositifs (113) de capture d'images configurés pour capturer des données d'image ; et
un ou plusieurs dispositifs informatiques configurés pour mettre en œuvre le procédé selon l'une quelconque des revendications 1 à 10.

12. Programme informatique qui, lorsqu'il est exécuté sur un ou plusieurs dispositifs informatiques, est configuré pour amener les un ou plusieurs dispositifs informatiques à réaliser le procédé selon l'une quelconque des revendications 1 à 10.

13. Mémoire non transitoire sur laquelle est stocké le programme informatique selon la revendication 12.
